# EUROPÄISCHE PATENTSCHRIFT

(11) **EP 3 773 064 B1**
(45) Veröffentlichungstag und Bekanntmachung des Hinweises auf die Patenterteilung: **13.03.2024**
(21) Anmeldenummer: 18782088.1
(22) Anmeldetag: 10.10.2018
(51) Int. Cl.: A45D 34/00, A61J 1/20, A61M 35/00, B65D 83/00

(54) **APPLIKATOR**
APPLICATOR
APPLICATEUR

(30) Priorität: 04.06.2018 WO PCT/EP2018/064643
(43) Veröffentlichungstag der Anmeldung: 17.02.2021
(73) Patentinhaber: Gemü GmbH, 6032 Emmen (CH)
(72) Erfinder: BISLIN, Raimund, 6233 Büron (CH); STRASSER, Daniel, 9200 Gossau (CH)
(74) Vertreter: Troesch Scheidegger Werner AG
(86) Internationale Anmeldenummer: PCT/EP2018/077620
(87) Internationale Veröffentlichungsnummer: WO 2019/233617

(56) Entgegenhaltungen:
- EP-A- 0 477 563
- WO-A1-94/13339
- WO-A1-97/14460
- WO-A1-97/14460
- WO-A1-2011/026198
- DE-A1- 4 021 697
- DE-C- 65 383
- DE-C- 519 479
- DE-U1- 20 218 493
- GB-A- 804 095
- RU-C1- 2 035 920
- US-A1- 2006 222 450

## Beschreibung

### TECHNISCHES GEBIET

Die vorliegende Erfindung betrifft einen Applikator und ein Set mit Applikator und Durchstechflasche.

### STAND DER TECHNIK

Aus dem Stand der Technik sind dicht verschliessbare zylinderförmige Behälter bekannt, auch Durchstechflaschen oder Vials genannt, welche zusammen mit einem Applikator verwendet werden können, um eine in der Durchstechflasche enthaltene Flüssigkeit, eine Paste oder ein Gas zu applizieren.

Die DE 200 22 899 offenbart einen zylinderförmigen Behälter mit einem geraden Boden, welcher mit einer Flüssigkeit gefüllt werden kann und welcher stirnseitig mit einer Membran dicht verschlossen werden kann. Der Behälter ist in einem Führungszylinder eines Applikators entlang einer Längsachse führbar. Ein Kolben ist konzentrisch zum Führungszylinder innerhalb diesem angeordnet. Der Kolben umfasst an seinem gegen den Behälter gerichteten Ende eine Spitze, mit welcher die Membran durchstossen werden kann. Ein durchgehender Kanal führt von der Spitze des Kolbens entlang der Längsachse zu einer Aussenseite des Applikators. Werden der Behälter und der Applikator zusammengeschoben, so durchstösst die Kolbenspitze die Membran. Durch das weitere zusammenschieben wird die im Behälter enthaltene Flüssigkeit durch den Kanal zur Aussenseite des Applikators gedrückt. Aufgrund des geraden Behälterbodens und der spitzen Ausgestaltung des Kolbens ist die im Behälter verbleibende Restmenge an Flüssigkeit bei vollständig zusammengeschobenem Behälter und Applikator relativ gross.

Die WO 2016/083451 offenbart einen Behälter mit einem spitz zulaufenden Boden, dessen Geometrie im Wesentlichen derjenigen der Kolbenspitze entspricht. Dadurch lässt sich die verbleibende Restflüssigkeit verringern. Bei einer solchen Ausgestaltung ist der grösste Anteil der Restflüssigkeit, d.h. der Flüssigkeit, die nicht appliziert werden kann, im Kanal des Kolbens enthalten.

Um das Kanalvolumen möglichst klein zu halten, sind die Kanäle mit einem möglichst kleinen Durchmesser ausgeführt. Kleine, respektive dünne Kanäle sind im Spritzgussverfahren nur sehr schwer oder gar nicht herzustellen oder müssen nachträglich mit sehr feinen Bohrern hergestellt werden, was den Fertigungsaufwand stark erhöht.

Das Dokument EP 0 477 563 A offenbart einen zylinderförmigen Behälter, der in einem doppelwandigen Halter angeordnet ist, wobei ein doppelwandiger Applikator mit einem in den Behälter ragenden Stössel auf dem Halter aufgesetzt ist und wobei die Innenseiten der beiden Wände des Applikators auf den jeweiligen Aussenseiten der beiden Wände des Halters geführt sind.

### BESCHREIBUNG DER ERFINDUNG

Eine Aufgabe der vorliegenden Erfindung besteht darin, einen Applikator bereitzustellen, welcher die obengenannten Nachteile vermeidet, welcher einfach zu fertigen ist und welcher zuverlässig und sicher funktioniert.

Diese Aufgabe wird durch einen Applikator mit den Merkmalen des Anspruchs 1 gelöst. Weitere Ausführungsformen des Applikators und eines Sets mit einem Applikator und einer Durchstechflasche, sind durch die Merkmale von weiteren Ansprüchen definiert.

Ein erfindungsgemässer Applikator umfasst eine zylindrische Hülse und einen Stössel, welche sich beide entlang einer gemeinsamen Längsachse erstrecken. Die Hülse umfasst eine erste Stirnseite und eine der ersten Stirnseite gegenüberliegende zweite Stirnseite. An der zweiten Stirnseite ist auf der Innenseite der Hülse ein zylindrischer erster Absatz ausgebildet, mit welchem die Hülse an einem zylindrischen Behälter anordbar ist. Anschliessend an den ersten Absatz ist ein zylindrischer zweiter Absatz ausgebildet, in welchem ein dazu komplementärer zylindrischer Kolben des Stössels verschiebbar angeordnet ist. Ein Kanal ist vorgesehen, welcher sich entlang der Längsachse durch den gesamten Stössel erstreckt. An der ersten Stirnseite ist auf der Innenseite der Hülse ein zylindrischer dritter Absatz ausgebildet, in welchem ein dazu komplementärer zylindrischer Schaft des Stössels verschiebbar angeordnet ist. Eine solche Ausgestaltung hat den Vorteil, dass der Kolben in der Hülse dichtend führbar ist. Zudem kann ein solcher Applikator mit geringen Anpassungen zusammen mit einer Vielzahl von Durchstechflaschen verwendet werden. Mit nur zwei Bauteilen ist diese Konstruktion einfach und zuverlässig. Beispielsweise sind die beiden Komponenten aus Kunststoff gefertigt. Ein solcher Applikator lässt sich im Spritzgussverfahren herstellen, insbesondere in einem Montage-Spritzgussverfahren, bei welchem die beiden Teile gemeinsam und gleichzeitig in einem einzigen Spritzguss-Vorgang im montierten Zustand hergestellt werden. Die Hülse und der Stössel können aus dem gleichen Material oder aus unterschiedlichen Materialine gefertigt sein. Die Hülse und der Stössel können eine unterschiedliche Härte aufweisen. Beispielsweise kann die Hülse einen harten Kunststoff umfassen und der Stössel einen weichen Kunststoff. In einem solchen Fall können die Einzelteile in einem Mehrkomponenten-Montage-Spritzguss gemeinsam hergestellt werden.

In einer Ausführungsform ist der Durchmesser des ersten Absatzes grösser als der Durchmesser des zweiten Absatzes und der Durchmesser des zweiten Absatzes ist grösser als der Durchmesser des dritten Absatzes. Die kaskadenförmige Abstufung der Absätze erlaubt eine einfache Herstellung der Hülse und des Stössels.

In einer Ausführungsform umfasst eine umlaufende Mantelfläche des ersten Absatzes ein erstes Verschlusselement, mit welchem die Hülse am Behälter befestigbar ist. Ein solches Verschlusselement kann eine Raste, einen Bajonettverschluss, einen Gewindeabschnitt oder ein Gewinde umfassen. Alternativ kann der Durchmesser der umlaufenden Mantelfläche des Absatzes der Hülse etwas kleiner ausgestaltet sein als der Aussendurchmesser des Behälters, wodurch die Hülse auf den Behälter geklemmt werden kann.

In einer Ausführungsform ist an einer umlaufenden ersten Stirnfläche des ersten Absatzes eine Dichtung vorgesehen. Die Dichtung kann gemeinsam einstückig mit der Hülse ausgebildet sein. Beispielsweise kann die Dichtung zusammen mit der Hülse im Mehrkomponenten-Spritzguss hergestellt werden. Alternativ kann ein Dichtring oder eine Dichtscheibe im ersten Absatz angeordnet sein.

In einer Ausführungsform ist an der zweiten Stirnseite eine Anfasung vorgesehen, welche an den ersten Absatz angrenzt. Die Anfasung erleichtert das Einführen des Stössels in die Hülse. Zudem kann die Anfasung Teil einer Rastausnehmung sein, wie dies später beschrieben wird. An der Mantelfläche des zweiten Absatzes kann ebenfalls eine Anfasung vorgesehen sein, welche an den ersten Absatz angrenzt. Diese Anfasung erleichterst ebenfalls das Einführen des Stössels in die Hülse beim Zusammenbauen.

In einer Ausführungsform ist an der ersten Stirnseite mindestens eine Raste vorgesehen, welche nach innen gerichtet über die Mantelfläche des dritten Absatzes ragt und in mindestens einer Nut lösbar verrastbar ist, welche im Schaft des Stössels ausgebildet ist. Das Verrasten dient als Transportsicherung. Zudem ist es vorteilhaft, wenn vor dem Durchstossen der Folie der Durchstechflasche eine Kraftschwelle überschritten werden muss und das Durchstossen daher ruckartig erfolgt. Durch die Dicke und den Dickenverlauf der Raste, ist die zur Überwindung der Verrastung benötigte Kraft einstellbar.

In einer Ausführungsform ist die mindestens eine Raste eine Lasche, welche sich in der Richtung der Längsachse winklig von der dritten Ausnehmung weg erstreckt. Beispielsweise erstreckt sie sich schräg nach innen. Durch die Breite der Laschen und durch deren Anzahl, ist die zur Überwindung der Verrastung benötigte Kraft einstellbar.

In einer Ausführungsform sind zwei oder mehr Rasten vorgesehen, welche gleichmässig oder ungleichmässig verteilt am Umfang der ersten Stirnseite angeordnet sind. Am äusseren Umfang der Hülse kann im Bereich des dritten Absatzes mindestens eine Rippe vorgesehen sein, welche sich radial nach aussen erstreckt. Diese Bauweise ist stabil, haptisch angenehm und erlaubt eine vorteilhafte Fertigung mittels Spritzgusses. Die die mindestens eine Rippe kann fluchtend mit der ersten Stirnseite und mit dem äusseren Umfang des Bereichs des zweiten Absatzes ausgebildet sein. Es können zwei oder mehr Rippen vorgesehen sein, welche gleichmässig oder ungleichmässig verteilt am Umfang der ersten Stirnseite (angeordnet sind. Die Rippen können gemeinsam einstückig mit dem Rest der Hülse ausgebildet sein.

Der Schaft und der Kolben sind gemeinsam einstückig miteinander ausgebildet, wobei der Kolben anschliessend an einen Endbereich des Schaftes angeordnet ist. Der Kolben umfasst eine umlaufende Mantelfläche und einen Boden, wobei der Boden, auf der dem Schaft gegenüberliegenden Seite des Kolbens angeordnet ist.

In einer Ausführungsform umfasst die Kolbenmantelfläche an ihrem dem Schaft zugewandten Rand eine geschlossen umlaufende Dichtnase, welche sich nach aussen über die Kolbenmantelfläche erstreckt. Die Dichtnase kann auch am Rand der Kolbenmantelfläche angeordnet sein, welcher dem Schaft abgewandt ist. Die Dichtnase kann auch zwischen den beiden Rändern an der Kolbenmantelfläche angeordnet sein. Die Kolbenmantelfläche kann auch mehrere Dichtnasen umfassen, welche bezüglich der Längsachse beabstandet zueinander angeordnet sind.

Der Boden des Kolbens ist konisch nach innen zulaufend ausgebildet, und der Kanal mündet in das Zentrum des Bodens. Der Winkel, der im Querschnitt durch den Kolbenboden und eine zur Längsachse senkrechte Fläche aufgespannt wird, kann kleiner als 45 Grad, 45 Grad oder grösser als 45 Grad ausgebildet sein. Beispielsweise kann der Winkel 30 Grad bis 80 Grad betragen. Beispielsweise kann der Winkel 40 Grad bis 60 Grad betragen. Alternativ kann der Kolbenboden auch flach ausgebildet sein.

An der Kante der kolbenseitigen Öffnung des Kanals sind zwei oder mehr Durchstosselemente vorgesehen, welche vom Boden des Kolbens abstehen. Mit den Durchstosselementen kann das Öffnen der Durchstechflasche verbessert werden, indem die zum Durchstossen der Folie benötigte Kraft reduziert wird und indem das Durchstossen derart erfolgt, dass sich keine Teile von der Folie ablösen, welche in den Kanal gelangen können und entweder den Kanal verstopfen oder zusammen mit dem zu applizierenden Medium ausgetragen werden. Beispielsweise ist das mindestens eine Durchstosselement zahnförmig ausgebildet.

Die zwei oder mehr Durchstosselemente sind gleichmässig oder ungleichmässig an der Kante der kolbenseitigen Öffnung des Kanals angeordnet. Beispielsweise können mehrere aneinander anschliessende Zähne vorgesehen sein. Die Zähne können auch beabstandet zueinander angeordnet sein. Beispielsweise sind drei Durchstosselemente vorgesehen, welche jeweils 90 Grad versetzt zueinander an der Kante angeordnet sind. Da auf einer Seite des Kanalrandes kein Durchstosselement vorgesehen ist, wird die Folie dort nicht Durchstossen. Dementsprechend wird die Folie um diesen Bereich aufgeklappt, wodurch verhindert wird, dass die Folie in den Kanal gelangt.

In einer Ausführungsform sind zwischen den Durchstosselementen Entlüftungsnuten vorgesehen, welche sich vom Kanal aus radial nach aussen in der Richtung des Kolbenmantels über einen Teilbereich des Kolbenbodens erstrecken. Die sich in der Durchstechflasche befindliche Restluft kann mit den Entlüftungsnuten zum Kanal geführt werden.

In einer Ausführungsform ist im Kolben eine Entlastungsnut vorgesehen, welche sich ringförmig von einer Schaft-nahen Stirnseite des Kolbens im Bereich der Kolbenmantelfläche entlang der Längsachse erstreckt. Durch die Entlastungsnut entsteht ein krempenförmiger Kolben, wodurch die Steifigkeit der Kolbenmantelfläche reduziert wird und sich besser der Hülse, bzw. der Durchstechflasche anpassen kann. Je dünner die verbleibende Wandstärke des Kolbens ist, desto nachgiebiger ist die Kolbenmantelfläche. Beispielsweise kann die radiale Dicke der Entlastungsnut im Wesentlichen der verbleibenden Wandstärke des Kolbens im Bereich der Kolbenmantelfläche entsprechen.

In einer Ausführungsform umfasst der Applikator ein Griffstück mit einem Schaft mit einem durchgehenden Kanal, welche sich beide entlang der Längsachse erstrecken, wobei das Griffstück mit seinem Anschluss am dazu komplementären Anschluss des Stössels lösbar befestigbar ist. An einem dem Kolben gegenüberliegenden Ende des Stössels kann ein Anschluss für das Griffstück vorgesehen sein.

In einer Ausführungsform umfasst das Griffstück mindestens einen Griff, welcher seitlich vom Schaft absteht und welcher derart ausgestaltet ist, dass mindestens die Fingerkuppe eines Benutzers daran anlegbar ist. Es können auch mehrere Griffe am Griffstück vorgesehen sein. Beispielsweise können zwei Griffe vorgesehen sein, welche bezüglich der Längsachse gegenüberliegend angeordnet sind. Anstelle mehrerer voneinander getrennter Griffe, kann auch eine umlaufend geschlossene Scheibe vorgesehen sein. Der oder die Griffe können derart ausgestaltet sein, dass mehrere Fingerkuppen gleichzeitig daran angreifen können.

In einer Ausführungsform umfasst der Applikator einen Aufsatz mit einem Schaft mit einem durchgehenden Kanal, welche sich beide entlang der Längsachse erstrecken, wobei der Aufsatz mit seinem Anschluss am dazu komplementären Anschluss des Griffstücks lösbar befestigbar ist. Der Aufsatz kann je nach der vorgesehenen Anwendung ausgestaltet sein. Beispielsweise kann er kurz oder lang sein. Alternativ oder zusätzlich kann er als Sprayaufsatz ausgebildet sein.

In einer Ausführungsform umfassen die Anschlüsse Absätze, welche Verbindungspaare bilden, wobei der männliche Partner des jeweiligen Verbindungspaares eine Erhebung mit einem kleineren Durchmesser als derjenige des entsprechenden Schaftes aufweist und wobei der weibliche Partner des jeweiligen Verbindungspaares eine Ausnehmung mit einem kleineren Durchmesser als derjenige des entsprechenden Schaftes aufweist. Beispielsweise umfasst der Stössel einen männlichen Anschluss, wobei das Griffstück an seinem einen Ende einen dazu komplementären weiblichen Anschluss umfasst und an seinem anderen Ende einen männlichen Anschluss umfasst, und wobei der Aufsatz einen dazu komplementären weiblichen Anschluss umfasst.

In einer Ausführungsform umfassen die Absätze der Verbindungspaare jeweils eine Raste, welche in einer dazu komplementären Nut lösbar verrastbar ist. Es können mehrere zueinander beabstandete Rasten und/oder Nuten vorgesehen sein.

In einer Ausführungsform sind die Absätze der Verbindungspaare gerade oder konisch ausgebildet.

In einer Ausführungsform umfassen die konischen Absätze der Verbindungspaare einen Luer-Konus oder eine Luer-Lock Verbindung.

In einer Ausführungsform ist der Winkel des Kolbenbodens derart bemessen, dass das Volumen, welches zwischen dem Kolbenboden und der Hülse eingeschlossen ist, wenn die Spitze des Bodens fluchtend mit der ersten Stirnseite der Hülse ausgerichtet ist, im Wesentlichen dem Volumen aller Kanäle des Applikators entspricht. Durch diese Ausgestaltung entspricht das Volumen, welches zwischen dem Kolbenboden und der Oberfläche des in der Durchstechflasche befindlichen Mediums eingeschlossen ist, wenn die Spitze des Bodens fluchtend mit der Mediumoberfläche ausgerichtet ist. Demnach kann das in den Kanälen befindliche Medium durch dieses Volumen im Wesentlichen vollständig durch die Kanäle gefördert werden, wodurch sich der im Applikator verbleibende Rest des zu applizierenden Mediums reduziert.

Die erwähnten Ausführungsformen des Applikators lassen sich in beliebiger Kombination einsetzen, sofern sie sich nicht widersprechen.

Ein erfindungsgemässes Set umfasst einen Applikator nach Anspruch 1 und einen Behälter, wobei der Behälter eine Durchstechflasche ist, einen Boden und eine an den Boden anschliessende und geschlossen umlaufende zylindrische Wand umfasst, welche sich entlang der Längsachse erstreckt und deren Öffnung mit einer Folie dicht verschliessbar ist. Der Applikator lässt sich selbstverständlich auch zusammen mit anderen Behältern verwenden, in welchen sich ein zu applizierendes Medium befindet.

In einer Ausführungsform ist die Aussenwand des Behälters fluchtend mit dem ersten Absatz der Hülse ausgebildet. Dadurch wird die Hülse auf dem Behälter zentriert und ausgerichtet.

In einer Ausführungsform ist an der Aussenwand des Behälters ein zweites Verschlusselement vorgesehen, welches mit dem dazu komplementären ersten Verschlusselement eine formschlüssige und/oder kraftschlüssige Verbindung bildet. Das zweite Verschlusselement kann beispielsweise ein Gewinde, ein Gewindeabschnitt oder eine Raste sein.

In einer Ausführungsform ist die Innenwand des Behälters fluchtend mit dem zweiten Absatz der Hülse ausgebildet ist. Somit ist ein im Wesentlichen stufenloser Übergang zwischen der Hülse und dem Behälter realisierbar, wodurch der Stössel gleichermassen gut in der Hülse wie im Behälter führbar ist.

In einer Ausführungsform ist an der Innenseite der Behälteröffnung eine Anfasung ausgebildet. Durch diese Anfasung lässt sich der Stössel leichter in den Behälter einführen.

In einer Ausführungsform bildet die Anfasung des Behälters alleine oder zusammen mit der Anfasung der Hülse eine Rastausnehmung. Die Abmessungen der Anfasung des Behälters im Kontaktbereich des Behälters mit der Hülse entsprechen im Wesentlichen den Abmessungen der Anfasung der Hülse. Die Dichtnase des Stössels ist lösbar in der Rastausnehmung verrastbar. Wenn die Dichtnase des Stössels in der Rastausnehmung verrastet ist, so ist der Kolben des Stössels im Wesentlichen vollständig im Behälter eingeführt und die Spitze des Kolbenbodens ist zumindest teilweise im zu applizierenden Medium eingetaucht. Der Benutzer muss eine vorgegebene Kraft überwinden, um den Stössel weiter in den Behälter einfahren zu können. Sobald die aufgewendete Kraft grösser ist als die zur Überwindung der Verrastung notwendige, wird das zu applizierende Medium stossartig durch die Kanäle gefördert und dementsprechend appliziert.

Die erwähnten Ausführungsformen des Sets lassen sich in beliebiger Kombination einsetzen, sofern sie sich nicht widersprechen.

Ein nicht beanspruchtes Verfahren zur Verwendung eines Sets gemäss einer der obigen Ausführungsformen umfasst die Schritte:
- Bereitstellen eines Behälters gemäss einer der obigen Ausführungsformen;
- Bereitstellen eines Applikators gemäss einer der obigen Ausführungsformen;
- Aufsetzen des Applikators über der mit Folie verschlossenen Öffnung des Behälters;
- Verschieben des Stössels entlang der Längsachse gegen den Boden des Behälters;
- Durchstossen der Folie.

In einer Ausführungsform umfasst das Verfahren den Schritt:
- Anbringen eines Griffstücks am Anschluss des Stössels.

In einer Ausführungsform umfasst das Verfahren den Schritt:
- Anbringen eines Aufsatzes am Anschluss des Griffstücks.

In einer Ausführungsform umfasst das Verfahren die Schritte:
- Dichten mit der Dichtnase des Stössels gegenüber dem zweiten Absatz der Hülse;
- Dichten mit der Dichtnase des Stössels gegenüber einer Innenfläche der Wand des Behälters.

In einer Ausführungsform umfasst das Verfahren den Schritt:
- Lösbares Verrasten der Dichtnase des Stössels der Rastausnehmung der Hülse und/oder des Behälters.

Die erwähnten Ausführungsformen des Verfahrens lassen sich in beliebiger Kombination einsetzen, sofern sie sich nicht widersprechen.

### KURZE BESCHREIBUNG DER FIGUREN

Ausführungsbeispiele der vorliegenden Erfindung werden nachstehend anhand von Figuren noch näher erläutert. Diese dienen lediglich zur Erläuterung und sind nicht einschränkend auszulegen. Es zeigen
Fig. 1 eine perspektivische Darstellung eines erfindungsgemässen Applikators, welcher auf einem Behälter aufgesetzt ist;
Fig. 2 eine Schnittansicht durch die Anordnung der Figur 1;
Fig. 3 eine perspektivische Darstellung des Stössels der Figur 1;
Fig. 4 eine Unteransicht des Stössels der Figur 3; und
Fig. 5 eine Schnittansicht durch den Stössel der Figur 4.

### DETAILLIERTE BESCHREIBUNG DER ERFINDUNG

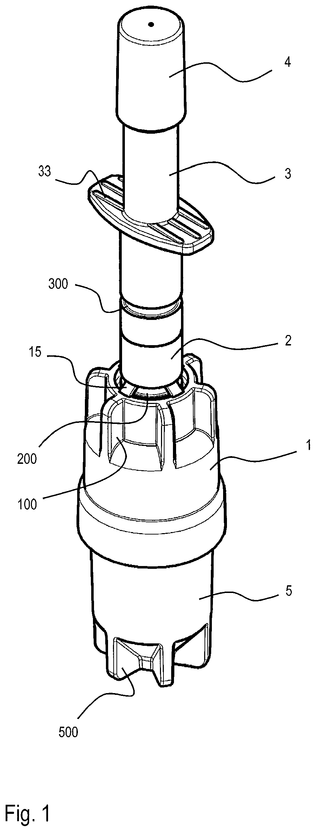

Die Figur 1 zeigt eine perspektivische Darstellung eines erfindungsgemässen Applikators, welcher auf einem Behälter 5 aufgesetzt ist. Der Applikator umfasst eine Hülse 1, eine Stössel 2, ein Griffstück 3 und einen Aufsatz 4.

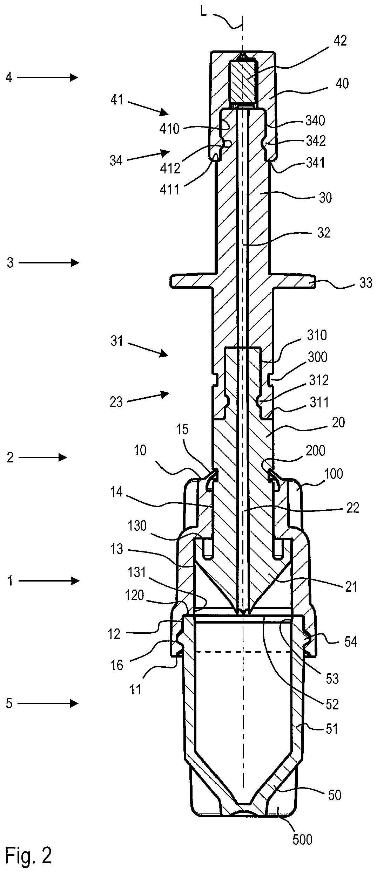

Die Figur 2 zeigt eine Schnittansicht durch die Anordnung der Figur 1. Die Hülse 1 erstreckt sich entlang einer Längsachse L und umfasst ein eine erste Stirnseite 10 und eine dieser gegenüberliegende zweite Stirnseite 11. Ein erster Absatz 12 erstreckt sich auf der Innenseite der Hülse 1 von der zweiten Stirnseite 11 an in die Richtung der ersten Stirnseite 10. Anschliessend an den ersten Absatz 12 ist ein zweiter Absatz 13 ausgebildet, dessen Durchmesser kleiner ausgebildet ist als derjenige des ersten Absatzes 12, wodurch beim ersten Absatz 12 eine ringförmig umlaufende erste Stirnfläche 120 gebildet wird. Im ersten Absatz 12 ist ein erstes Verschlusselement 16 in der Form eines Gewindes vorgesehen. An der gegen den ersten Absatz 12 hin gerichteten Kante des zweiten Absatzes 13 ist eine Anfasung 131 ausgebildet. In der dargestellten Ausführungsform ist die Anfasung 0,7 Millimeter breit und weist einen Winkel von 37 Grad auf. Anschliessend an den zweiten Absatz 13 ist ein dritter Absatz 14 ausgebildet, dessen Durchmesser kleiner ausgebildet ist als derjenige des zweiten Absatzes 13, wodurch beim zweiten Absatz 13 eine ringförmig umlaufende zweite Stirnfläche 130 gebildet wird. An der ersten Stirnseite 10 der Hülse 1 sind Rasten 15 vorgesehen, welche sich von einer inneren Kante der ersten Stirnseite 10 aus schräg nach innen gegen die Längsachse L von der zweiten Stirnseite 11 weg erstrecken. In der dargestellten Ausführungsform sind vier am inneren Umfang der zweiten Stirnseite 11 gleichmässig verteilte Rasten 15 vorgesehen. Die dargestellten Rasten 15 sind 1 Millimeter breit und 1 Millimeter lang. Die Wandstärke der Hülse 1 ist im Bereich des ersten Absatzes 12 am dünnsten, im Bereich des dritten Absatzes 14 etwas dicker und im Bereich des zweiten Absatzes 13 am dicksten. Die Aussenkontur der Hülse ist ergonomisch mit einem Anzug von 1 Grad nach oben abnehmend ausgebildet. Daraus ergeben sich in der dargestellten Ausführungsform eine Wandstärke von 0,5 Millimeter im Bereich des ersten Absatzes, welcher eine Länge von 1,8 Millimeter aufweist, eine Wandstärke von 1,3 Millimeter im Bereich des zweiten Absatzes, welcher eine Länge von 6 Millimeter aufweist und eine Wandstärke von 1,2 Millimeter im Bereich des dritten Absatzes, welcher eine Länge von 3,5 Millimeter aufweist. An der Aussenseite der Hülse 1, im Bereich des dritten Absatzes 14, sind Rippen 100 vorgesehen, welche fluchtend mit der Aussenfläche der Hülse 1 im Bereich des zweiten Absatzes 13 ausgebildet sind. In der dargestellten Ausführungsform sind sechs über den Umfang gleichmässig verteilte Rippen 100 vorgesehen. Ein Stössel 2 ist entlang der Längsachse L verschiebbar im zweiten Absatz 13 und im dritten Absatz 14 der Hülse 1 angeordnet.

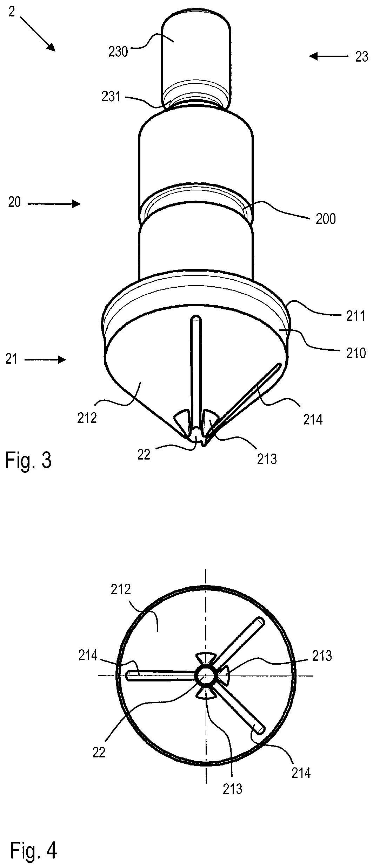

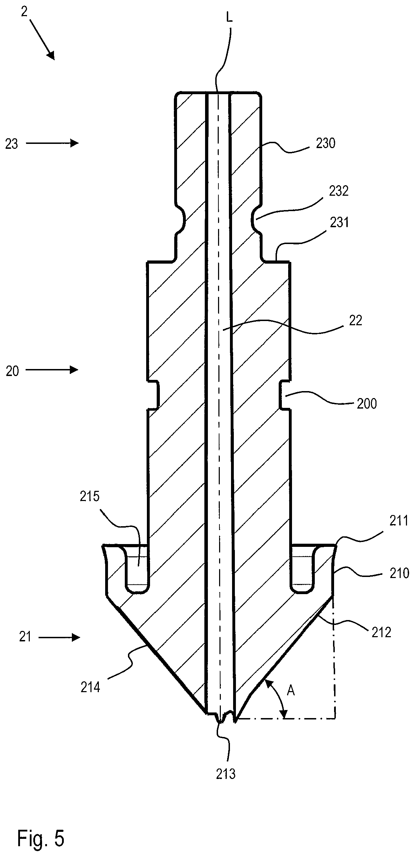

Die Figur 3 zeigt eine perspektivische Darstellung des Stössels 2 der Figur 1, die Figur 4 zeigt eine Unteransicht des Stössels 2 der Figur 3 und die Figur 5 zeigt eine Schnittansicht durch den Stössel 2 der Figur 4. Der Stössel 2 umfasst einen Schaft 20, welcher sich zylindrisch entlang der Längsachse L erstreckt. In der dargestellten Ausführungsform ist der Schaft 12 Millimeter lang und umfasst einen Durchmesser von 5 Millimeter. Der Durchmesser des Schaftes 20 des Stössels 2 entspricht dem Durchmesser des dritten Absatzes 14 der Hülse 1, wodurch sich im zusammengesetzten Zustand eine sichere Führung des Stössels 2 in der Hülse 1 ergibt. An einer ersten Seite des Schaftes 20 ist ein Kolben 21 ausgebildet und an einer der ersten Seite gegenüberliegenden zweiten Seite des Schaftes 20 ist ein Anschluss 23 ausgebildet. Der Kolben 21 umfasst eine zylindrische Kolbenmantelfläche 210 mit einer Dichtnase 211, welche an der gegen den Anschluss 23 gerichteten Kante der Kolbenmantelfläche 210 ausgebildet ist. Der Durchmesser der Kolbenmantelfläche 210 entspricht dem Durchmesser des zweiten Absatzes 13 der Hülse 1, wodurch sich im Zusammenbau eine sichere Führung des Kolbens 21 in der Hülse 1 ergibt. Die Dichtnase 211 erstreckt sich geschlossen über den ganzen Umfang der Kolbenmantelfläche 210 und ragt von dieser radial nach aussen über diese hinaus. Im zusammengebauten Zustand wird die Dichtnase 211 durch den zweiten Absatz 13 zusammengedrückt, wodurch sich die Dichtwirkung erhöht.

Auf der dem Anschluss 23 abgewandten Seite des Kolbens 21 ist ein Kolbenboden 212 ausgebildet, welcher sich entlang der Längsachse L konisch verjüngt. In der dargestellten Ausführungsform misst der Winkel A, welcher durch die Kontur des Kolbenbodens 212 im Querschnitt und einer zur Längsachse L senkrechte Ebene aufgespannt wird 50 Grad. Der Stössel 2 umfasst einen durchgehenden Kanal 22, welcher sich von der Spitze des Kolbenbodens 212 durch den Schaft 20 bis zum Anschluss 23 und durch diesen hindurch erstreckt. An der Austrittskante des Kanals 22 beim Kolbenboden 212 sind drei Durchstosselemente 213 vorgesehen, welche am Umfang der Austrittskante jeweils unter einem Winkel von 90 Grad zueinander versetzt angeordnet sind. D.h. in drei von vier Quadranten des Kolbenbodens 212 ist jeweils ein Durchstosselement 213 in der Form eines Durchstosszahnes angeordnet. Jeweils in der Mitte zwischen zwei benachbarten Zähnen 213 ist eine Entlüftungsnut 214 angeordnet. Jede Entlüftungsnut 214 erstreckt sich von der Austrittskante des Kanals 22 im Wesentlichen radial nach aussen gegen die Kolbenmantelfläche 210 des Kolbens 21 über einen Teilbereich des Kolbenbodens 212. Das seitliche Ende jeder Entlüftungsnut 214 ist beabstandet zur gemeinsamen Kante des Kolbenbodens 212 und der Kolbenmantelfläche 210. Im Kolben 21 des Stössels 2 ist eine Ausgleichsnut 215 ausgebildet, welche sich ringförmig in der Verlängerung des Schaftes 20 erstreckt, d.h. die Innenseite der Nut 215 wird durch den Schaft 20 gebildet und die Aussenseite durch eine zum Schaft 20 konzentrische Ringfläche. Dementsprechend ergibt sich in der dargestellten Ausführungsform ein krempenförmiger Kolbenmantel mit einer Wandstärke von 1,7 Millimeter. Der Kolbenmantel weist einen Durchmesser von 8 Millimeter auf und ist 1,8 Millimeter hoch. Im Schaft 20 ist beabstandet zum Kolben 21 eine vollständig umlaufende Nut 200 ausgebildet, in welche die Rasten 15 der Hülse 1 verrastbar sind. Der Abstand zwischen dem Kolben 21 und der Nut 200 entspricht dem Abstand zwischen der zweiten Stirnfläche 130 des zweiten Absatzes 13 der Hülse 1 und deren Rasten 15. Der Anschluss 23 des Stössels 2 umfasst eine Absatz 230, in welchem eine geschlossen umlaufende Nut 232 ausgebildet ist. Der Durchmesser des Absatzes 230 ist kleiner als derjenige des Schaftes 20, wodurch sich eine geschlossen umlaufende ringförmige Stirnseite 231 ergibt. Die Nut 232 des Anschlusses 23 ist beabstandet zur Stirnseite 231 ausgebildet. Am Anschluss 23 des Stössels 2 kann ein Griffstück 3 mit einem entsprechenden Anschluss 31 angeordnet werden. Das Griffstück 3 umfasst einen Schaft 30, welcher sich entlang der Längsachse L erstreckt, wobei an seinem einen Ende ein Anschluss 31 für den Stössel 2 vorgesehen ist und an seinem anderen Ende ein Anschluss 34 für einen Aufsatz 4. Ein durchgehender Kanal 32 erstreckt sich entlang der Längsachse L durch das ganze Griffstück 3. Der Anschluss 31 für den Stössel umfasst einen Absatz 310, in welchem eine geschlossen umlaufende Raste 312 ausgebildet ist. Der Absatz 310 ist als zentrische Bohrung ausgebildet, deren Durchmesser demjenigen des Anschlusses 23 des Stössels 2 entspricht. Der verbleibende Teil des Griffstücks 3 um den Absatz 310 ergibt eine geschlossen umlaufende Stirnseite 311, welche im zusammengebauten Zustand an der entsprechenden Stirnseite 231 des Stössels 2 anschlagen kann. Die umlaufende Raste 312 des Griffstücks 3 ist derart beabstandet zur Stirnseite 311 angeordnet, dass sie im Zusammenbau in der entsprechenden Nut 232 des Stössels 2 verrasten kann. An der Aussenseite des Schafts 30 des Griffstücks 3 ist beabstandet zur Stirnseite 311 eine geschlossen umlaufende Nut 300 ausgebildet. Die dargestellte Nut ist 1 Millimeter breit und 0,3 Millimeter tief. Die Tiefe kann jedoch bis 0,6 Millimeter betragen. Der Abstand zwischen der Nut 200 des Stössels 2 und der Nut 300 des Griffstücks 3 entspricht im Wesentlichen dem Abstand zwischen der Vorderkante des Kolbenmantels des Stössels 2 und der Oberfläche des zu applizierenden Mediums im Behälter 5 im Zusammenbau. Beabstandet zur Nut 300 des Griffstücks 3 sind Griffe 33 am Schaft 30 angeordnet. Die Griffe 33 stehen seitlich derart vom Schaft 30 ab, dass zumindest jeweils eine Fingerkuppe daran anlegbar ist. Auf der dem Stössel 2 abgewandten Seite des Griffstücks 3 ist ein Anschluss 34 für einen Aufsatz 4 vorgesehen. Der Anschluss 34 umfasst einen Absatz 340 und eine darin ausgebildete vollständig umlaufende Nut 342. Der Durchmesser des Absatzes 340 ist kleiner als der Durchmesser des Schafts 30 des Griffstücks 3, wodurch sich ein bolzenförmiger Anschluss 34 mit einer ringförmigen Stirnseite 341 ergibt. Die Nut 342 ist beabstandet zur Stirnseite 341 ausgebildet. Am Anschluss 34 des Griffstücks 3 ist im Zusammenbau der Aufsatz 4 mit einem dazu komplementären Anschluss 41 angeordnet. Da Aufsatz 4 umfasst einen Schaft 40, welcher sich entlang der Längsachse L erstreckt und in dessen Innern sich ein Kanal 42 über die gesamte Länge erstreckt. Der Anschluss 41 des Aufsatzes 4 umfasst einen Absatz 410 an welchem eine vollständig umlaufende Raste 412 ausgebildet ist, welche im Zusammenbau in die entsprechende Nut 342 des Griffstücks 3 eingreift. Der Absatz 410 ist Teil einer zentrischen Bohrung im Schaft 40. Die verbleibende Fläche um die Bohrung bildet eine ringförmige Stirnseite 411, welche im Zusammenbau an der entsprechenden Stirnseite 341 des Griffstücks 3 anschlagen kann. In der dargestellten Ausführungsform ist der Aufsatz 4 ein Sprühdüsenaufsatz, mit welchem das zu applizierende Medium zerstäubt werden kann. Im Zusammenbau ist ein Behälter 5 im ersten Absatz 12 der Hülse 1 aufgenommen. In der dargestellten Ausführungsform ist der Behälter 5 eine Durchstechflasche mit einem Boden 50 und einer an den Boden 50 anschliessenden geschlossen umlaufenden Wand 51. Die Öffnung der Durchstechflasche 5 ist durch eine Folie 52 dicht verschlossen. An der inneren Kante der Öffnung ist eine Anfasung 53 ausgebildet. Auf der Aussenseite der Wand 51 ist ein zweites Verschlusselement 54 beabstandet zur Öffnung angeordnet; Dargestellt ist ein Gewinde. Der Aussendurchmesser der Wand 51 entspricht dem Innendurchmesser des ersten Absatzes 12 der Hülse 1 und der Innendurchmesser der Wand 51 entspricht dem Innendurchmesser des zweiten Absatzes 13 der Hülse 1. Der Boden 50 ist konisch ausgebildet, wobei die Konizität des Bodens 50 derjenigen des Kolbens 21 des Stössels 2 entspricht. Am Boden 50 sind Rippen 500 angeordnet, welche sich vom konischen Teil des Bodens 50 in der Richtung der Längsachse L bis zur Spitze des Bodens erstrecken und welche sich seitlich bis zur Aussenseite der Wand 51 erstrecken. In der dargestellten Ausführungsform sind sechs Rippen 500 vorgesehen, welche gleichmässig verteilt bezüglich des Umfangs des Behälters 5 angeordnet sind.

### BEZUGSZEICHENLISTE

| | | | |
|---|---|---|---|
| 1 | Hülse | 22 | Kanal |
| 10 | erste Stirnseite | 23 | Anschluss |
| 100 | Rippe | 230 | Absatz |
| 11 | zweite Stirnseite | 231 | Stirnseite |
| 12 | erster Absatz | 232 | Nut |
| 120 | erste Stirnfläche | 3 | Griffstück |
| 13 | zweiter Absatz | 30 | Schaft |
| 130 | zweite Stirnfläche | 300 | Nut |
| 131 | Anfasung | 31 | Anschluss |
| 14 | dritter Absatz | 310 | Absatz |
| 15 | Raste | 311 | Stirnseite |
| 16 | erstes Verschlusselement | 312 | Raste |
| | | 32 | Kanal |
| 2 | Stössel | 33 | Griff |
| 20 | Schaft | 34 | Anschluss |
| 200 | Nut | 340 | Absatz |
| 21 | Kolben | 341 | Stirnseite |
| 210 | Kolbenmantelfläche | 342 | Raste |
| 211 | Dichtnase | 4 | Aufsatz |
| 212 | Kolbenboden | 40 | Schaft |
| 213 | Durchstosselement | 41 | Anschluss |
| 214 | Entlüftungsnut | 410 | Absatz |
| 215 | Ausgleichsnut | | |
| 411 | Stirnseite | 52 | Folie |
| 412 | Raste | 53 | Anfasung |
| 42 | Kanal | 54 | zweites Verschlusselement |
| 5 | Behälter | | |
| 50 | Boden | L | Längsachse |
| 500 | Rippe | A | Winkel |
| 51 | Wand | | |

## Patentansprüche

1. Ein Applikator umfassend eine zylindrische Hülse (1) und einen Stössel (2), welche sich beide entlang einer gemeinsamen Längsachse (L) erstrecken, wobei die Hülse (1) eine erste Stirnseite (10) umfasst und eine der ersten Stirnseite (10) gegenüberliegende zweite Stirnseite (11) umfasst, wobei an der zweiten Stirnseite (11) auf der Innenseite der Hülse (1) ein zylindrischer erster Absatz (12) ausgebildet ist, mit welchem die Hülse (1) an einem zylindrischen Behälter (5) anordbar ist, wobei anschliessend an den ersten Absatz (12) ein zylindrischer zweiter Absatz (13) ausgebildet ist, in welchem ein dazu komplementärer zylindrischer Kolben (21) des Stössels (2) verschiebbar angeordnet ist, wobei ein Kanal (22) vorgesehen ist, welcher sich entlang der Längsachse (L) durch den gesamten Stössel (2) erstreckt, wobei an der ersten Stirnseite (10) auf der Innenseite der Hülse (1) ein zylindrischer dritter Absatz (14) ausgebildet ist, in welchem ein dazu komplementärer zylindrischer Schaft (20) des Stössels (2) verschiebbar angeordnet ist, wobei der Schaft (20) und der Kolben (21) gemeinsam einstückig miteinander ausgebildet sind, wobei der Kolben (21) anschliessend an einen Endbereich des Schaftes (20) angeordnet ist, wobei der Kolben (21) eine umlaufende Mantelfläche (210) und einen Boden (212) umfasst, wobei der Boden (212) auf der dem Schaft (20) gegenüberliegenden Seite des Kolbens (21) angeordnet ist, wobei der Boden (212) des Kolbens (21) konisch nach innen zulaufenden ausgebildet ist, und wobei der Kanal (22) in das Zentrum des Bodens (212) mündet, **dadurch gekennzeichnet, dass** an einer Kante der kolbenseitigen Öffnung des Kanals (22) zwei oder mehr Durchstosselemente (213) vorgesehen sind, welche gleichmässig oder ungleichmässig an der Kante der kolbenseitigen Öffnung des Kanals (22) angeordnet sind und welche vom Boden (212) des Kolbens (21) abstehen.

2. Der Applikator gemäss Anspruch 1, wobei der Durchmesser des ersten Absatzes (12) grösser ist als der Durchmesser des zweiten Absatzes (13) und wobei der Durchmesser des zweiten Absatzes (13) grösser ist als der Durchmesser des dritten Absatzes (14).

3. Der Applikator gemäss Anspruch 1 oder 2, wobei eine umlaufende Mantelfläche des ersten Absatzes (12) ein erstes Verschlusselement (16) umfasst, mit welchem die Hülse (1) am Behälter (5) befestigbar ist.

4. Der Applikator gemäss einem der vorangehenden Ansprüche, wobei an einer umlaufenden ersten Stirnfläche (120) des ersten Absatzes (12) eine Dichtung vorgesehen ist.

5. Der Applikator gemäss einem der vorangehenden Ansprüche, wobei an der ersten Stirnseite (10) eine, zwei oder mehr Rasten (15) vorgesehen sind, welche gleichmässig oder ungleichmässig verteilt am Umfang der ersten Stirnseite (10) angeordnet sind und welche nach innen gerichtet über die Mantelfläche des dritten Absatzes (14) ragen und in mindestens einer Nut (200) lösbar verrastbar sind, welche im Schaft (20) des Stössels (2) ausgebildet ist.

6. Der Applikator gemäss Anspruch 5, wobei die eine, zwei oder mehr Rasten (15) Laschen sind, welche sich in der Richtung der Längsachse (L) winklig von der dritten Ausnehmung (14) weg erstrecken.

7. Der Applikator gemäss Anspruch 1, wobei die Kolbenmantelfläche (210) an ihrem dem Schaft (20) zugewandten Rand eine geschlossen umlaufende Dichtnase (211) umfasst, welche sich nach aussen über die Kolbenmantelfläche (210) erstreckt.

8. Der Applikator gemäss Anspruch 1, wobei zwischen den Durchstosselementen (213) Entlüftungsnuten (214) vorgesehen sind, welche sich vom Kanal (22) aus radial nach aussen in der Richtung des Kolbenmantels (210) über einen Teilbereich des Kolbenbodens (212) erstrecken.

9. Der Applikator gemäss einem der vorangehenden Ansprüche, wobei im Kolben (21) eine Entlastungsnut (215) vorgesehen ist, welche sich ringförmig von einer Schaft-nahen Stirnseite des Kolbens (21) im Bereich der Kolbenmantelfläche (210) entlang der Längsachse (L) erstreckt.

10. Der Applikator gemäss einem der vorangehenden Ansprüche, umfassend ein Griffstück (3) mit einem Schaft (30) mit einem durchgehenden Kanal (32), welche sich beide entlang der Längsachse (L) erstrecken, wobei das Griffstück (3) mit seinem Anschluss (31) am dazu komplementären Anschluss (23) des Stössels (2) lösbar befestigbar ist.

11. Der Applikator gemäss Anspruch 10, wobei das Griffstück (3) mindestens einen Griff (33) umfasst, welcher seitlich vom Schaft (30) absteht und welcher derart ausgestaltet ist, dass mindestens die Fingerkuppe eines Benutzers daran anlegbar ist.

12. Der Applikator gemäss einem der vorangehenden Ansprüche, umfassend einen Aufsatz (4) mit einem Schaft (40) mit einem durchgehenden Kanal (42), welche sich beide entlang der Längsachse (L) erstrecken, wobei der Aufsatz (4) mit seinem Anschluss (41) am dazu komplementären Anschluss (34) des Griffstücks (3) lösbar befestigbar ist.

13. Der Applikator gemäss Anspruch 12, wobei die Anschlüsse (23,31;34,41) Absätze (230,310;340,410) umfassen, welche Verbindungspaare bilden, wobei der männliche Partner des jeweiligen Verbindungspaares eine Erhebung mit einem kleineren Durchmesser als derjenige des entsprechenden Schaftes (20,30) aufweist und wobei der weibliche Partner des jeweiligen Verbindungspaares eine Ausnehmung mit einem kleineren Durchmesser als derjenige des entsprechenden Schaftes (30,40) aufweist.

14. Der Applikator gemäss Anspruch 13, wobei die Absätze (230,310;340,410) der Verbindungspaare jeweils eine Raste (312,342) umfassen, welche in einer dazu komplementären Nut (232,342) lösbar verrastbar ist.

15. Der Applikator gemäss Anspruch 13 oder 14, wobei die Absätze (230,310,340,410) der Verbindungspaare gerade oder konisch ausgebildet sind.

16. Der Applikator gemäss einem der Ansprüche 13 bis 15, wobei die konischen Absätze (230,310,340,410) der Verbindungspaare einen Luer-Konus oder eine Luer-Lock Verbindung umfassen.

17. Der Applikator gemäss einem der Ansprüche 8 bis 16, wobei der Winkel des Kolbenbodens (212) derart bemessen ist, dass das Volumen, welches zwischen dem Kolbenboden (212) und der Hülse (1) eingeschlossen ist, wenn die Spitze des Bodens (212) fluchtend mit der ersten Stirnseite (120) der Hülse (1) ausgerichtet ist, im Wesentlichen dem Volumen aller Kanäle (22,32,42) des Applikators entspricht.

18. Ein Set umfassend einen Applikator gemäss einem der vorangehenden Ansprüche und einen Behälter (5), wobei der Behälter (5) eine Durchstechflasche ist, einen Boden (50) und eine an den Boden (50) anschliessende und geschlossen umlaufende zylindrische Wand (51) umfasst, welche sich entlang der Längsachse (L) erstreckt und deren Öffnung mit einer Folie (52) dicht verschliessbar ist.

19. Das Set gemäss Anspruch 18, wobei die Aussenwand des Behälters (5) fluchtend mit dem ersten Absatz (12) der Hülse (1) ausgebildet ist.

20. Das Set gemäss Anspruch 18 oder 19, wobei an der Aussenwand des Behälters (5) ein zweites Verschlusselement (54) vorgesehen ist, welches mit dem dazu komplementären ersten Verschlusselement (16) eine formschlüssige und/oder kraftschlüssige Verbindung bildet.

21. Das Set gemäss einem der Ansprüche 18 bis 20, wobei die Innenwand des Behälters (5) fluchtend mit dem zweiten Absatz (13) der Hülse (1) ausgebildet ist.

22. Das Set gemäss einem der Ansprüche 18 bis 21, wobei an der Innenseite der Behälteröffnung eine Anfasung (53) ausgebildet ist.

23. Das Set gemäss Anspruch 22, wobei die Anfasung (53) des Behälters (5) alleine oder zusammen mit einer Anfasung (131) der Hülse (1), welche an den ersten Absatz (12) der Hülse (1) angrenzt, eine Rastausnehmung bilden, wobei die Abmessungen der Anfasung (53) des Behälters (5) im Kontaktbereich des Behälters (5) mit der Hülse (1) im Wesentlichen den Abmessungen der Anfasung (131) der Hülse (1) entsprechen und wobei die Dichtnase (211) des Stössels (2) lösbar in der Rastausnehmung verrastbar ist.

## Claims

1. An applicator comprising a cylindrical sleeve (1) and a plunger (2), both of which extend along a common longitudinal axis (L), wherein the sleeve (1) comprises a first end side (10) and a second end side (11), which is located opposite the first end side (10), wherein, at the second end side (11), a cylindrical first section (12) is formed on the inner side of the sleeve (1), by which the sleeve (1) can be arranged on a cylindrical container (5), wherein the first section (12) is followed by a cylindrical second section (13), in which a complementary cylindrical piston (21) of the plunger (2) is arranged in a displaceable manner, wherein a channel (22) is provided, which extends through the entire plunger (2) along the longitudinal axis (L), wherein at the first end side (10), a cylindrical third section (14) is formed on the inner side of the sleeve (1), in which a complementary cylindrical shaft (20) of the plunger (2) is arranged in a displaceable manner, wherein the shaft (20) and the piston (21) are jointly formed in one piece with one another, wherein the piston (21) is arranged adjoining an end region of the shaft (20), wherein the piston (21) comprises a circumferential lateral surface (210) and a bottom (212), wherein the bottom (212) is arranged on the side of the piston (21) opposite the shaft (20), wherein the bottom (212) of the piston (21) is designed to taper conically inwards, and wherein the channel (22) opens at the center of the bottom (212), **characterized in that** two or more piercing elements (213) are provided at the edge of the piston-side opening of the channel (22), which are regularly or irregularly arranged at the edge of the piston-side opening of the channel (22), and which project from the bottom (212) of the piston (21).

2. The applicator according to claim 1, wherein the diameter of the first section (12) is larger than the diameter of the second section (13), and wherein the diameter of the second section (13) is larger than the diameter of the third section (14) .

3. The applicator according to claim 1 or 2, wherein a circumferential lateral surface of the first section (12) comprises a first closure element (16), by which the sleeve (1) can be fastened to the container (5).

4. The applicator according to any one of the preceding claims, wherein a seal is provided on a circumferential first end face (120) of the first section (12).

5. The applicator according to any one of the preceding claims, wherein one, two or more catches (15) are provided at the first end side (10), which are arranged regularly or irregularly distributed around the circumference of the first end side (10), and which project inwards beyond the lateral surface of the third section (14) and can be releasably latched into at least one groove (200) that is formed in the shaft (20) of the plunger (2).

6. The applicator according to claim 5, wherein the one, two or more catches (15) are tabs that extend at an angle away from the third section (14) towards the longitudinal axis (L).

7. The applicator according to claim 1, wherein the piston's lateral surface (210) comprises, on the edge thereof facing towards the shaft (20), a sealing lug (211) that runs all the way round and that extends outwards beyond the piston's lateral surface (210).

8. The applicator according to claim 1, wherein venting grooves (214) are provided between the piercing elements (213), which venting grooves extend radially outwards from the channel (22) towards the piston skirt over part of the piston bottom (212).

9. The applicator according to any one of the preceding claims, wherein a relief groove (215) is provided in the piston (21), which relief groove, starting from an end side of the piston (21) close to the shaft, extends annularly along the longitudinal axis (L) in the region of the piston's lateral surface (210).

10. The applicator according to any one of the preceding claims, comprising a grip piece (3) having a shaft (30) with a continuous channel (32), both of which extend along the longitudinal axis (L), wherein the grip piece (3) can be detachably fastened by its connector (31) to the complementary connector (23) of the plunger (2).

11. The applicator according to claim 10, wherein the grip piece (3) comprises at least one grip (33), which projects laterally from the shaft (30) and which is configured such that at least the fingertip of a user can be placed thereon.

12. The applicator according to any one of the preceding claims, comprising an attachment (4) having a shaft (40) with a continuous channel (42), both of which extend along the longitudinal axis (L), wherein the attachment (4) can be detachably fastened by its connector (41) to the complementary connector (34) of the grip piece (3).

13. The applicator according to claim 12, wherein the connectors (23, 31; 34, 41) comprise sections (230, 310; 340, 410) that form connecting pairs, wherein the male partner of the respective connecting pair has a protrusion with a diameter smaller than that of the corresponding shaft (20, 30), and wherein the female partner of the respective connecting pair has a recess with a diameter smaller than that of the corresponding shaft (30, 40).

14. The applicator according to claim 13, wherein the sections (230, 310; 340, 410) of the connecting pairs each comprise a catch (312, 342), which can be releasably latched into a complementary groove (232, 342).

15. The applicator according to claim 13 or 14, wherein the sections (230, 310, 340, 410) of the connecting pairs are straight or conical.

16. The applicator according to any one of claims 13 to 15, wherein the conical sections (230, 310, 340, 410) of the connecting pairs comprise a Luer cone or a Luer lock connection.

17. The applicator according to any one of claims 8 to 16, wherein the angle of the piston bottom (212) is such that the volume enclosed between the piston bottom (212) and the sleeve (1) when the tip of the bottom (212) is in alignment with the first end face (120) of the sleeve (1) substantially corresponds to the volume of all the channels (22, 32, 42) of the applicator.

18. A set comprising an applicator according to any one of the preceding claims and a container (5), wherein the container (5) is a vial and comprises a bottom (50) and a cylindrical wall (51) adjoining the bottom (50) and running all the way round the latter, which wall extends along the longitudinal axis (L) and the opening of which can be tightly closed by a film (52).

19. The set according to claim 18, wherein the outer wall of the container (5) is in alignment with the first section (12) of the sleeve (1).

20. The set according to claim 18 or 19, wherein a second closure element (54) is provided on the outer wall of the container (5) and establishes a form-fitting and/or force-fitting connection with the complementary first closure element (16).

21. The set according to any one of claims 18 to 20, wherein the inner wall of the container (5) is in alignment with the second section (13) of the sleeve (1).

22. The set according to any one of claims 18 to 21, wherein a chamfer (53) is formed on the inner side of the container opening.

23. The set according to claim 22, wherein the chamfer (53) of the container (5), alone or together with a chamfer (131) of the sleeve (1), which is adjacent to the first section (12) of the sleeve (1), forms a latching recess, wherein the dimensions of the chamfer (53) of the container (5) in the region where the container (5) makes contact with the sleeve (1) substantially correspond to the dimensions of the chamfer (131) of the sleeve (1), and wherein the sealing lug (211) of the plunger (2) can be releasably latched into the latching recess.

## Revendications

1. Un applicateur comprenant un manchon cylindrique (1) et un poussoir (2), qui s'étendent tous deux le long d'un axe longitudinal commun (L), le manchon (1) comprenant une première face frontale (10) et une deuxième face frontale (11) opposée à la première face frontale (10), un premier épaulement cylindrique (12) étant formé sur la deuxième face frontale (11) sur la face intérieure du manchon (1), avec lequel le manchon (1) peut être disposé sur un récipient cylindrique (5), un deuxième épaulement cylindrique (13) étant formé à la suite du premier épaulement (12), dans lequel un piston cylindrique complémentaire (21) du poussoir (2) est disposé de manière à pouvoir coulisser, un canal (22) étant prévu, lequel s'étend le long de l'axe longitudinal (L) à travers l'ensemble du poussoir (2), un troisième épaulement cylindrique (14) étant formé sur la première face frontal (10) sur le côté intérieur du manchon (1), dans lequel une tige cylindrique (20) complémentaire du poussoir (2) est disposée de manière à pouvoir coulisser, la tige (20) et le piston (21) étant formés ensemble d'un seul pièce, dans lequel le piston (21) étant disposé à la suite d'une zone d'extrémité de la tige (20), le piston (21) comprenant une surface d'enveloppe périphérique (210) et un fond (212), le fond (212) étant disposé sur le côté du piston (21) opposé à la tige (20), le fond (212) du piston (21) étant réalisé de manière à se terminer en cône vers l'intérieur, et le canal (22) débouchant au centre du fond (212), **caractérisé en ce que** deux ou plusieurs éléments de perforation (213) sont prévus sur un bord de l'ouverture du canal (22) côté piston, qui sont disposés de manière régulière ou irrégulière sur le bord de l'ouverture du canal (22) côté piston et qui font saillie du fond (212) du piston (21).

2. L'applicateur selon la revendication 1, dans lequel le diamètre du premier épaulement (12) est plus grand que le diamètre du deuxième épaulement (13) et dans lequel le diamètre du deuxième épaulement (13) est plus grand que le diamètre du troisième épaulement (14).

3. L'applicateur selon la revendication 1 ou 2, dans lequel une surface d'enveloppe périphérique du premier épaulement (12) comprend un premier élément de fermeture (16) avec lequel le manchon (1) peut être fixé au récipient (5) .

4. L'applicateur selon l'une des revendications précédentes, dans lequel un joint d'étanchéité est prévu sur une première surface frontale périphérique (120) du premier épaulement (12).

5. L'applicateur selon l'une des revendications précédentes, dans lequel il est prévu sur la première face frontale (10) un, deux ou plusieurs crans d'arrêt (15) qui sont répartis de manière régulière ou irrégulière sur la périphérie de la première face frontale (10) et qui font saillie vers l'intérieur au-delà de la surface d'enveloppe du troisième épaulement (14) et peuvent être encliquetés de manière amovible dans au moins une rainure (200) qui est formée dans la tige (20) du poussoir (2).

6. L'applicateur selon la revendication 5, dans lequel les un, deux ou plusieurs crans (15) sont des pattes qui s'étendent dans la direction de l'axe longitudinal (L) en s'éloignant angulairement du troisième épaulement (14).

7. L'applicateur selon la revendication 1, dans lequel la surface d'enveloppe de piston (210) comprend sur son bord tourné vers la tige (20) un nez d'étanchéité (211) périphérique fermé qui s'étend vers l'extérieur sur la surface d'enveloppe de piston (210).

8. L'applicateur selon la revendication 1, dans lequel des rainures d'aération (214) sont prévues entre les éléments de perforation (213), lesquelles s'étendent radialement vers l'extérieur à partir du canal (22) dans la direction de la surface d'enveloppe de piston (210) sur une zone partielle de la tête de piston (212).

9. L'applicateur selon l'une des revendications précédentes, dans lequel il est prévu dans le piston (21) une rainure de décharge (215) qui s'étend en forme d'anneau depuis une face frontale du piston (21) proche de la tige dans la zone de la surface d'enveloppe du piston (210) le long de l'axe longitudinal (L).

10. L'applicateur selon l'une des revendications précédentes, comprenant une pièce de préhension (3) avec une tige (30) dotée d'un canal traversant (32), qui s'étendent tous deux le long de l'axe longitudinal (L), la pièce de préhension (3) pouvant être fixée de manière amovible par son raccord (31) au raccord (23) du poussoir (2) qui lui est complémentaire.

11. L'applicateur selon la revendication 10, dans lequel la pièce de préhension (3) comprend au moins une poignée (33) qui dépasse latéralement de la tige (30) et qui est configurée de telle sorte qu'au moins le bout du doigt d'un utilisateur peut y être appliqué.

12. L'applicateur selon l'une des revendications précédentes, comprenant un embout (4) avec une tige (40) avec un canal traversant (42), qui s'étendent tous deux le long de l'axe longitudinal (L), l'embout (4) pouvant être fixé de manière amovible par son raccord (41) au raccord (34) complémentaire à celui-ci de la pièce de préhension (3) .

13. L'applicateur selon la revendication 12, dans lequel les raccords (23,31;34,41) comprennent des épaulements (230,310;340,410) formant des paires de connexion, le partenaire mâle de chaque paire de connexion présentant un bossage de diamètre inférieur à celui de la tige correspondante (20,30) et le partenaire femelle de chaque paire de connexion présentant un évidement de diamètre inférieur à celui de la tige correspondante (30,40).

14. L'applicateur selon la revendication 13, dans lequel les épaulements (230,310;340,410) des paires de connexion comprennent chacun un cran (312,342) qui peut être encliqueté de manière amovible dans une rainure (232,342) qui lui est complémentaire.

15. L'applicateur selon la revendication 13 ou 14, dans lequel les épaulements (230,310;340,410) des paires de connexion sont droits ou coniques.

16. L'applicateur selon l'une quelconque des revendications 13 à 15, dans lequel les épaulements coniques (230,310;340,410) des paires de raccords comprennent un cône Luer ou un raccord Luer-Lock.

17. L'applicateur selon l'une quelconque des revendications 8 à 16, dans lequel l'angle de la tête de piston (212) est tel que le volume enfermé entre la tête de piston (212) et le manchon (1), lorsque le sommet de la tête (212) est aligné avec la première surface frontale (120) du manchon (1), correspond sensiblement au volume de tous les canaux (22,32,42) de l'applicateur.

18. Un ensemble comprenant un applicateur selon l'une des revendications précédentes et un récipient (5), le récipient (5) étant un flacon perforateur, comprenant un fond (50) et une paroi cylindrique (51) se raccordant au fond (50) et faisant le tour de manière fermée, qui s'étend le long de l'axe longitudinal (L) et dont l'ouverture peut être fermée de manière étanche par un film (52).

19. L'ensemble selon la revendication 18, dans lequel la paroi extérieure du récipient (5) est alignée avec le premier épaulement (12) du manchon (1).

20. L'ensemble selon la revendication 18 ou 19, dans lequel un deuxième élément de fermeture (54) est prévu sur la paroi extérieure du récipient (5), lequel forme avec le premier élément de fermeture (16) qui lui est complémentaire une liaison par complémentarité de forme et/ou par adhérence.

21. L'ensemble selon l'une des revendications 18 à 20, dans lequel la paroi interne du récipient (5) est alignée avec le deuxième épaulement (13) du manchon (1).

22. L'ensemble selon l'une des revendications 18 à 21, dans lequel un chanfrein (53) est formé sur la face interne de l'ouverture du récipient.

23. L'ensemble selon la revendication 22, dans lequel le chanfrein (53) du récipient (5) forme, seul ou avec un chanfrein (131) du manchon (1), qui est adjacent au premier épaulement (12) du manchon (1), un évidement d'encliquetage, les dimensions du chanfrein (53) du récipient (5) dans la zone de contact du récipient (5) avec le manchon (1) correspondant essentiellement aux dimensions du chanfrein (131) du manchon (1) et le nez d'étanchéité (211) du poussoir (2) pouvant être encliqueté de manière amovible dans l'évidement d'encliquetage.
